# EUROPÄISCHE PATENTSCHRIFT

(11) **EP 0 544 273 B1**
(45) Veröffentlichungstag und Bekanntmachung des Hinweises auf die Patenterteilung: **02.09.1998**
(21) Anmeldenummer: 92120127.3
(22) Anmeldetag: 25.11.1992
(51) Int. Cl.: A61B 17/00, A61C 19/00, A61G 15/14

(54) **Ärztliche Behandlungseinrichtung, insbesondere für chirurgische Zwecke**
Medical treatment device, in particular for surgical purposes
Dispositif de traitement médical, en particulier à des fins chirurgicales

(30) Priorität: 25.11.1991 DE 4138681
(43) Veröffentlichungstag der Anmeldung: 02.06.1993
(73) Patentinhaber: KALTENBACH & VOIGT GMBH & CO., 88400 Biberach (DE)
(72) Erfinder: Strohmaier, Ernst, W-7953 Bad Schussenried (DE)
(74) Vertreter: Schmidt-Evers, Jürgen, Dipl.-Ing.

(56) Entgegenhaltungen:
- EP-A- 0 012 873
- EP-A- 0 183 972
- DE-A- 1 766 056

## Beschreibung

Die Erfindung bezieht sich auf eine Behandlungseinrichtung, wie sie aus dem Druckschrift DE-A-1766056 bekannt ist, nämlich auf eine Behandlungseinrichtung, die ein motorbetriebenes Behandlungsinstrument aufweist mit einem aus mindestens zwei Teilen bestehenden Handstück, von denen das hintere Handstückteil durch eine Medien-Versorgungsleitung mit einem Steuergerät verbindbar ist und das vordere Handstückteil zur Aufnahme eines Behandlungswerkzeugs dient, wobei das hintere Handstückteil eine über Kupplungsmitteln verbundene, separierbare Motorpatrone enthält.

Bei der Behandlung eines menschlichen oder tierischen Körpers ist eine Kontaminierung des Behandlungsinstrumentes mit vom Körper stammenden Krankheitserregern vorgegeben. Dies gilt sowohl für solche Behandlungsinstrumente, mit denen der Körper in einem Abstand behandelt werden kann, z.B. Sprühdüsen, und insbesondere für solche Behandlungsinstrumente, bei deren Benutzung ein unmittelbarer Kontakt mit dem Körper stattfindet. Mit einer Kontaminierung des Behandlungsinstruments ist insbesondere dann zu rechnen, wenn das Behandlungsinstrument mit Körperflüssigkeiten, z.B. Blut, in Kontakt kommt, wie es insbesondere bei einer Behandlung mit einem chirurgischen Behandlungsinstrument der Fall ist.

Es ist deshalb beabsichtigt, das Behandlungsinstrument einschließlich seines Versorgungsschlauches, durch den hindurch die erforderliche Antriebsenergie und ggfs. ein Behandlungsmittel, insbesondere ein Kühlmittel oder dergleichen zugeführt wird, zu sterilisieren.

Bei einem Behandlungsinstrument kann das Handstück aus einem Antriebsteil mit einem fest installierten Motor und einem Werkzeugträgerteil bestehen, wobei das Antriebsteil und das Werkzeugträgerteil lösbar miteinander kuppelbar sind. Es können somit verschiedene, für die jeweilige Behandlung prädestinierte Werkzeugträgerteile wahlweise mit dem Antrieb verbunden und wieder gelöst werden.

Zum Sterilisieren ist Dampfsterilisieren bis 3 bar Druck bei einer Temperatur von 145°C etwa üblich.

Für eine sterilisationsfeste Ausbildung des Motors ist eine aufwendige Ausgestaltung des Motors und des ihn aufnehmenden Aufnahmeteils am Antriebsteil erforderlich, da andernfalls Sterilisationsmittel in den Motor einzudringen vermag, der die vorhandenen Lager- und Funktionsteile beeinträchtigt und somit die Lebensdauer des Behandlungsinstruments wesentlich herabsetzt. Eine absolut dichte Ausführung des Motors gegen Eintritt von Sterilisationsmittel ist schwierig und aufwendig, so daß mit hohen Herstellungskosten zu rechnen ist.

Der Erfindung liegt die Aufgabe zugrunde, eine Behandlungseinrichtung der eingangs angegebenen Art so auszugestalten, daß der Motor für die Sterilisation des Behandlungsinstrumentes handhabungsfreundlich von diesem freigestellt werden kann.

Diese Aufgabe wird durch die Merkmale des Anspruchs 1 gelöst. Vorteilhafte Weiterbildungen der Erfindung sind in den Unteransprüchen beschrieben.

Beim erfindungsgemäßen Behandlungsinstrument ist eine Motorpatrone lösbar am Antriebsteil gekuppelt, und es ist im Bereich des Behandlungsplatzes eine Parkstelle mit einer Aufnahmevorrichtung für die Motorpatrone vorgesehen, auf den bzw. in die die Motorpatrone handhabungsfreundlich übergeben und wieder entnommen werden kann. Dabei braucht die Motorpatrone nicht direkt manuell ergriffen zu werden.

Beim Vorhandensein einer zwischen der Hülse des Handstücks und der Motorpatrone und zwischen letzterer und der Aufnahmevorrichtung vorhandenen lösbaren Kupplung ist es auf besonders gesicherter Weise möglich, die Motorpatrone mit dem Handstück an die Aufnahmevorrichtung zu übergeben und von dieser wieder zu übernehmen.

Da die Motorpatrone in der Hülse des Handstücks vorzugsweise geschlossen bzw. völlig verdeckt aufgenommen ist, ist eine Kontammination der Motorpatrone verhindert. Da außerdem die Motorpatrone ohne jede weitere manuelle Berührung vom zu behandelnden Körper entfernt am Parkplatz deponiert werden kann, ist eine sterile Handhabung der Motorpatrone gewährleistet, wobei das Handstück und der Versorgungsschlauch sterilisiert werden können. Danach wird die Motorpatrone mit dem zugehörigen Teil des sterilisierten Handstück übernommen und das Handstück einsatzfertig vervollständigt.

Im Rahmen der Erfindung ist es möglich, die Hülse des Handstücks am Antriebsteil oder am Werkzeugträgerteil vorzusehen. In beiden Fällen ist es möglich, die Motorpatrone ohne direkten im manuellen Zugriff mit dem entsprechenden Handstückteil am Parkplatz zu deponieren und wieder zu übernehmen.

Es ist aus der EP-PS 0 183972 zwar an sich bekannt, eine Motorpatrone für ein zahnärztliches Handstück vorzusehen, jedoch geht es bei dieser bekannten Ausgestaltung darum, ein und dasselbe Antriebssteil eines Handstücks an mehrere Werkzeugträgerteile unterschiedlicher Hersteller und deshalb unterschiedlicher Formen und Anschlußausführungen bei Gewährleistung einer Medienübertragung anschließen zu können. Bei dem einen Werkzeugträgerteil ist rückseitig eine Kupplungshülse vorgesehen, in die die Motorpatrone einschiebbar ist. Bei einem anderen Werkzeugträgerteil ist eine separate Kupplungshülse rückseitig an das Werkzeugträgerteil ankuppelbar, in die die Motorpatrone rückseitig einschiebbar ist, wobei die Kupplungshülse rückseitig an das vordere Ende des Antriebsteils anschließbar ist.

In den Unteransprüchen sind Merkmale enthalten, die die erfindungsgemäße Lösung des Problems weiter verbessern, einfache und kostengünstige Einzelausgestaltungen umfassen und die Handhabungsfreundlichkeit sowie Funktionssicherheit verbessern.

Das erfindungsgemäße Behandlungsinstrument enthält insbesondere ein Handstück, insbesondere für eine vorliegende Behandlungseinrichtung, das aus zwei lösbar miteinander kuppelbaren Instrumententeilen besteht, zwischen denen ein Antrieb in Form einer Motorpatrone lösbar kuppelbar ist, um sie zu trennen und z.B. nicht zu sterilisieren.

Nachfolgend werden die Erfindung und weitere durch sie erzielbare Vorteile anhand bevorzugter Ausführungsbeispiele und einer Zeichnung näher erläutert.

Es zeigt
Fig. 1 eine erfindungsgemäße ärztliche Behandlungseinrichtung mit einem Behandlungsinstrument und einer damit durch eine Versorgungsleitung verbundenen Steuereinrichtung für einen ärztlichen Behandlungsplatz in perspektivischer Darstellung;
Fig. 2 bis 4 die in Figur 1 mit X gekennzeichneten Einzelheit in vergrößerten perspektivischen Darstellungen und verschiedenen Funktionsstellungen;
Fig. 5 das Antriebsteil des zweistückigen Behandlungsinstruments mit zugehöriger Versorgungsleitung im Längsschnitt;
Fig. 6 und 7 eine Parkstelle mit einer Aufnahmevorrichtung für eine Motorpatrone des Handstücks in zwei verschiedenen Funktionsstellungen;
Fig. 8 die in Fig. 6 mit Y gekennzeichnete Einzelheit in vergrößerter Darstellung.

Die Hauptteile der Behandlungseinrichtung 1 sind ein Behandlungsinstrument in Form eines Handstücks 2 und ein Steuergerät 3 mit Steuertasten an seiner Frontseite und mit einer integrierten elektronischen Steuereinrichtung, mit der das Handstück 2 durch eine biegsame bzw. flexible Versorgungsleitung 4 verbunden ist. Das Handstück 2 trägt an seinem vorderen Ende ein Behandlungswerkzeug 5, das vorzugsweise gegen wahlweise erforderliche unterschiedliche Werkzeuge austauschbar ist. Bei solchen Behandlungsinstrumenten, bei denen die Zuführung eines Kühlmittels, insbesondere einer Kühlflüssigkeit wie eine Salzlösung (NaCl), zur Behandlungsstelle erforderlich ist, ist dem Steuergerät 3 eine Kühlmittelpumpe 6 zugeordnet, die vorzugsweise am Steuergerät 3 angebaut oder in diese integriert sein kann. Die zum vorderen Ende des Handstücks 2 führende Kühlmittelleitung 7 verläuft wenigstens teilweies durch die Versorgungsleitung 4 bzw. das Handstück 2. Am Steuergerät 3 ist eine Steckkupplung 8 angeordnet, in die ein am freien Ende der Versorgungsleitung 4 angeordneter Stecker 9 einsteckbar ist zwecks Anschluß an die elektronische Steuereinrichtung. Das Steuergerät 3 kann auf einem Gestell oder Tisch 11 stehen. Es ist eine Haltevorrichtung 12 mit einem Ablageteil für das Handstück 2 in dessen Bereitschaftsstellung vorzugsweise am Steuergerät 3 angeordnet. Bei der vorliegenden Ausgestaltung ist ein Ablageteil in Form einer muldenförmigen schräg, zum Behandlungsplatz hin geneigten Schale oder Platte 13 vorgesehen, auf der das Handstück 2 in einer mit dem Werkzeug 5 schräg nach unten weisenden Position formschlüssig aufliegt. Das Handstück 2 ist aufgrund der muldenförmigen Form der Platte 13 gegen seitliches Verlagern gesichert. Vorzugsweise ist der Muldengrund an die taillierte Form des Handstücks 2 angepaßt, so daß sich auch in dessen Längsrichtung eine formschlüssige Halterung ergibt. Die Platte 13 ist an einem etwa horizontalen vom Tisch 11 oder vom Gehäuse des Steuergeräts 3 wegragenden Tragarm 12a gehalten, an dessen Ende sie vorzugsweise in einem Gelenk um eine etwa vertikale Schwenkachse frei schwenkbar gelagert ist.

Der Tisch 11 steht mit Rollen (nicht dargestellt) auf dem Boden und ist durch manuellen Zugriff, vorzugsweise an einem frontseitigen Handgriff 11a verschiebbar. Der Tisch 11 ruht auf dem oberen Ende einer Tragsäule 11b, die auf dem Stegteil 10a eines U-förmigen Standrahmens 10 steht, dessen Schenkelteile 10b zur Bedienungsseite hin weisen.

Der Tragarm 12a ist längenveränderlich und an seinem der Haltevorrichtung 12 abgewandten Ende in einem Gelenk mit vertikaler Schwenkachse mit dem Steuergerät 3 verbunden. Aufgrund dieser Ausgestaltung ist die Haltevorrichtung 12 mit dem Handstück 2 sowohl horizontal frei schwenkbar als auch bezüglich ihres Abstandes zum Steuergerät 3 einstellbar. Hierdurch ist sie in einem großen Bewegungsbereich frei verstellbar und in bezüglich des Behandlungsplatzes oder der Behandlungsstelle günstigen, wahlweisen Bereitshaftsstellungen einstellbar, in denen die behandelnde Person das Handstück handhabungsfreundlich ergreifen und wieder ablegen kann. In der Zeichnung ist der Tragarm 12a mit gestrichelten Linien in seiner ausgezogenen Position dargestellt. Bei dieser andeutungsweisen Darstellung fehlt die Haltevorrichtung 12, um das sie tragende Gelenk 16 am freien Ende des Tragarms 12a zu zeigen, daß durch eine vertikale Bohrung gebildet sein kann, in der ein von der Haltevorrichtung 12 nach unten ragender runder Zapfen (nicht dargestellt) von oben mit Bewegungsspiel einsteckbar ist.

Im Rahmen der Erfindung kann es sich bei dem Tragarm 12a um einen teleskopierbaren Tragarm handeln. Bei der vorliegenden Ausgestaltung ist der Tragarm 12a im Sinne eines Kniehebels mit zwei Tragarmteilen 12b, 12c ausgebildet, die an ihren einander zugewandten Enden in einem mittleren Gelenk 18 mit vertikaler Schwenkachse miteinander verbunden sind. Vorzugsweise liegen die beiden miteinander verbundenen Enden der Tragarmteile 12b, 12c aufeinander, wobei sie sich überlappen und von einem Gelenkbolzen in vertikalen Bohrungen durchsetzt sind. Es ist zum einen wegen Raumersparnis und zum anderen aus Gründen hinreichende Stabilität von Vorteil, die Tragarmteile 12b, 12c aus flachen Leisten rechteckigen Querschnitts zu bilden, die flach angeordnet sind.

Es ist von Vorteil, den Tragarm 12a so zu lagern, daß er in seiner eingeschobenen bzw. eingeschwenkten Stellung eine möglichst dichte Anordnung der Platte 13 am Steuergerät 3 ermöglicht. Dabei ist es möglich, den Tragarm 12a oberhalb des Steuergeräts 3 anzuordnen bzw. zu lagern, um ihn oberhalb des Steuergeräts 3 in dessen vertikalen Projektionsbereich ggfs. mit der Haltevorrichtung 12 einschwenken zu können, wodurch eine sehr raumsparende Anordnung geschaffen ist. Es ist auch möglich, den Tragarm 12a in einem unter dem Steuergerät vorhandenen Freiraum (nicht dargestellt) anzuordnen, bzw zu lagern, wobei ein solcher Freiraum durch Distanzteile zwischen dem Steuergerät 3 und dem Tisch 11 geschaffen werden könnte. Bei der vorliegenden Ausgestaltung ist im unteren Bereich des kastenförmigen Steuergeräts, hier unterhalb der Bedienungs- bzw. Tastaturfläche, eine horizontale Ausnehmung 21 vorgesehen, in die der Tragarm 12a hineinschwenkbar ist. Dabei kann das Gelenk im Bereich der Frontseite des Steuergeräts 3 oder innerhalb der Ausnehmung 21 angeordnet sein, wie es in der Zeichnung dargestellt ist. Die Ausnehmung 21 ist bei der vorliegenden Ausgestaltung im vorderen linken Eckenbereich des Steuergeräts 3 angeordnet, und sie ist sowohl zur Bedienungsseite, d.h. von vorne und von der linken Seite zugänglich bzw. offen. Die sich von der linken Seite des Steuergeräts 3 nach rechts erstreckende Breite b der Ausnehmung 21 entspricht in etwa einem dreiviertel der Breite B des Steuergeräts 3, so daß die Ausnehmung 21 in einem Abstand von der rechten Seite des Steuergeräts 3 endet. In der eingeschwenkten Stellung befindet sich die Platte 13 unmittelbar vor der rechten Frontseitenhälfte des Steuergeräts 3. Die Höhe der Ausnehmung 21 ist so groß bemessen, daß der Tragarm 12a mit vertikalem Bewegungsspiel einschwenkbar ist.

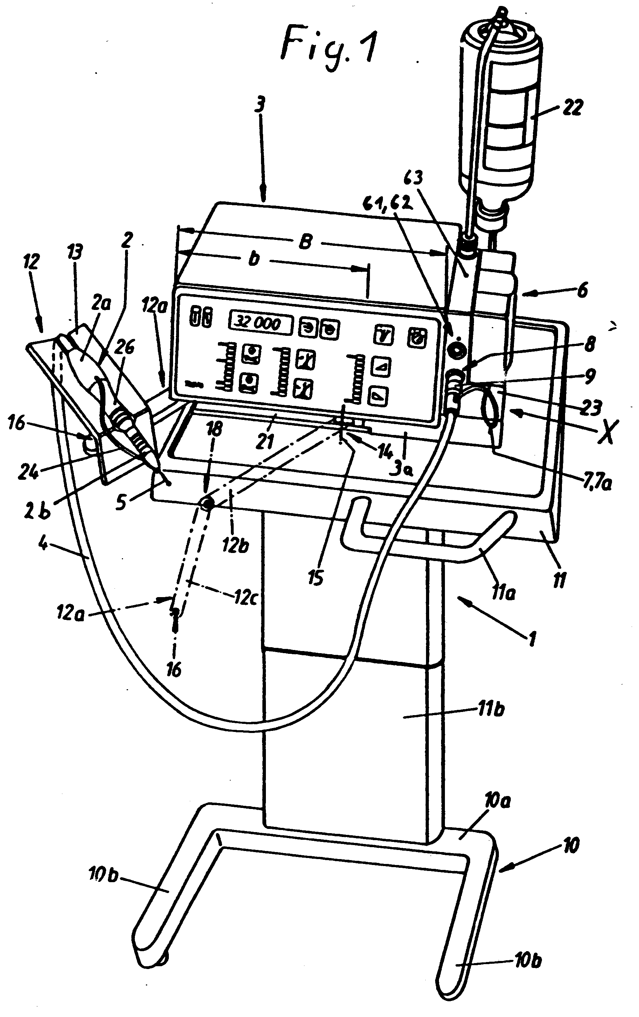

Vorzugsweise sind die Breite b der Ausnehmung 21 und die Länge des inneren Tragarmteils 12b so aneinander angepaßt, daß in der eingeschwenkten Stellung gem. Figur 1 das innere Tragarmteil 12c bzw. das mittlere Gelenk 18 nicht seitlich aus der Ausnehmung 21 herausragen.

Vorzugsweise ist die Ausnehmung 21 in einem insbesondere plattenförmigen Untersatz, hier in einer Grundplatte 3a des kastenförmigen Steuergerätes 3 ausgebildet. Die Grundplatte 3a ist lösbar am Steuergerät 3 befestigbar z.B. durch Schrauben. Die Vorrichtung bzw. das Steuergerät 3 ist somit durch die ansetzbare Grundplatte 3a ggfs. mit der Haltevorrichtung 12 modular ergänzbar oder wahlweise nachrüstbar.

Die vorbeschriebene Anordnung und Ausgestaltung der Haltevorrichtung 12 ermöglicht eine wahlweise Einstellung der Bereitschaftsstellung des Handstücks 2 im gesamten Frontbereich des Steuergerätes 3. Die auszuwählende Bereitschaftsstellung der Haltevorrichtung 12 kann somit an eine Vielzahl und auch schwierige Behandlungspositionen angepaßt werden.

Das Handstück 2 besteht aus zwei in seiner Längsrichtung hintereinander angeordneten Teilen, nämlich einem an der Versorgungsleitung 4 montierten Antriebsteil 2a und einem an dessen vorderen Ende durch eine Schnellkupplung lösbar befestigbaren Werkzeugträgerteil 2b. Im Antriebsteil 2a ist ein geeigneter Antriebmotor z.B. ein Elektromotor oder ein druckluftbetriebener Turbinenmotor angeordnet, der in einem Gehäuse in Form einer Motorpatrone 14 kartuschenförmig gekappselt ist. Das Antriebsteil 2a weist zur Aufnahme der Motorpatrone 14 eine vorderseitig offene zylindrische Hülse 15 auf, die so lang bemessen ist, daß sie wenigstens das Gehäuse der Motorpatrone 14 vollständig überdeckt. An der Rückseite der Motorpatrone 14 sind vorzugsweise diametral gegenüberliegend zwei Kontaktelemente, insbesondere in Form von Steckkupplungen 16 vorgesehen, die mit zwei Kontaktelementen in Form vom Hülsengrund achsparallel vorstehenden Kontaktstiften 17 korrespondieren. Die Kontaktstifte 17 stecken in einem im Querschnitt runden Gehäuse-Einsatzstück 18, das in einem am hinteren Ende der Hülse 15 angeordneten Ringbund 19 sitzt und durch eine den von hinten vorragenden Ringbund 19 radial in einem Loch durchfassende Schraube gesichert ist. Das Einsatzstück 18 besteht aus elektrisch nicht leitendem Material, insbesondere Kunstsoff. Vom Einsatzstück 18 ragt außerdem ein Positioniertift axparallel vor, der in ein entsprechendes Positionierloch 22 am rückseitigen Ende der Motorpatrone 14 paßt. Der Positionierstift 21 ist bezüglich den Kontaktstiften 17 in Umfangsrichtung versetzt angeordnet und zwecks Verdeutlichung in die Schnittebene verlegt, so daß nur ein Kontaktstift 17 sichtbar ist. Das Einsatzstück 18 weist rückseitig einen verjüngten zylindrischen Ansatz 23 auf, auf dem ein biegsamer Schutzschlauch 24 aufgesteckt und durch eine Schlauchklemme 24a oder dergleichen gesichert ist. Im Schutzschlauch 24 erstrecken sich je nach Ausführung mehrere Leiter z.B. zwei elektrische Kabel 25, dessen Adern in nicht dargestellter Weise mit den Kontaktstiften 17 verbunden sind, ein Kühlmittellschlauch 7a und ggfs ein Lichtleiter, alle in flexibler Ausführung.

Das Antriebsteil 2a weist zwei zueinander koaxial angeordnete Gehäuseteile auf, nämlich zum einen die Hülse 15 und eine bezüglich dieser rückseitige Hülsenkappe 26, die den hinteren Endbereich oder die hintere Hälfte der Hülse 15 übergreift, das Einsatzstück 18 überdeckt und sich bis in den freien Endbereich des Ansatzes 23 erstreckt, wo sie den Schutzschlauch 24 mit Bewegungsspiel umgibt. Die Hülsenkappe 26 weist in ihrem etwa mittleren Bereich einen Innenringansatz 27 mit einer zylindrischen Innenfläche 28 auf, die vorzugsweise gegenüber der zylindrischen Innenfläche 29 des Ringansatzes 19 im Durchmesser etwas kleiner bemessen ist. Im hinteren Bereich des Einsatzstücks 18 ist zwischen letzterem und dem Ringansatz 27 ein Innengwinde/Außengenwinde vorgesehen, mit dem Hülsenkappe 26 von hinten aufschraubbar ist. Das vordere Ende der im Querschnitt runden Hülsenkappe 26 sitzt auf einem zylindrischen Außenringansatz 31 und ist durch einen in einer Nut vorzugsweise im Außenringansatz 31 angeordneter Dichtungsring (O-Ring) abgedichtet. Auch die Innenflächen des Ringansatzes 19 und Innenringansatzes 27 sind durch jeweils einen O-Ring abgedichtet, der in einer Nut im Einsatzstück 18 sitzt. Hinter dem vorderen freien Randbereich der Hülsenkappe 26 befindet sich ein weiterer Innenringansatz 33, der von einer sich schräg nach vorne und außen erstreckenden Bohrung durchsetzt ist, in dem ein Röhrchen 34 mit seinem hinteren Ende fest und dicht eingesetzt ist, das außerhalb der Hülsenkappe 26 etwa axparallel nach vorne abgewinkelt ist. Zwischen dem Außenringansatz 31 und dem Innenringansatz 27 erstreckt sich zwischen der Hülse 15 und der Hülsenkappe 26 ein freier Ringraum 35, von dem das Röhrchen 34 ausgeht und der einen Abschnitt der Kühlmediumleitung 7 bildet. Letztere ist durch einen axial und einen radial verlaufenden Kanalabschnitt 36, 37 im Einsatzstück 18 mit dem Ringraum 35 verbunden.

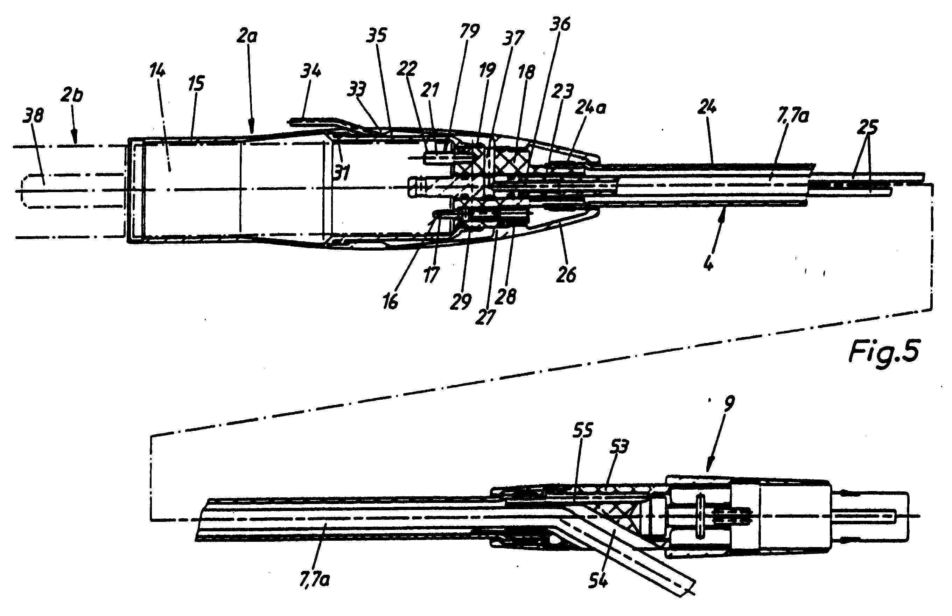

Wie am besten die Figuren 5, 6 und 8 zeigen, weist die Motorpatrone 14 einen koaxialen vorderen Hülsenzapfen 38 auf, der mit einem rückseitigen Flansch 39 an die Motorpatrone 14 vorzugsweise mittels axparallelen Schrauben angeflanscht ist.

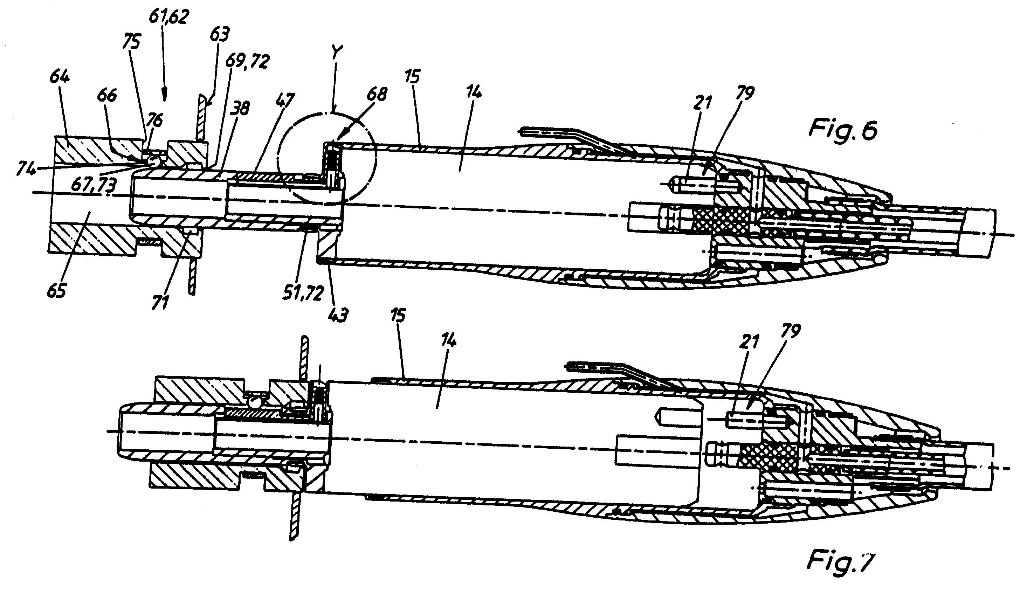

Die Motorpatrone 14 ist in der Hülse 15 in ihrer vorzugsweise vollends eingeschobenen Position axial lösbar verriegelbar. Wie insbesondere aus den Figuren 6 und 8 zu entnehmen ist, dient hierzu ein im Flansch 39 radial verschiebbar in einer Bohrung 40 in Flansch 39 gelagerter Riegelbolzen 41, der durch eine Feder 42 radial nach außen beaufschlagt ist und in eine am vorderen Ende der Hülse 15 angeordnete Ringnut 43 einzurasten vermag. Zwecks Erleichterung des Einrastens weist der Riegelbolzen 41 rückseitig eine Anlaufschräge 44 auf. Die Feder 42 ist vorzugsweise eine Druckfeder, die in einer radial nach außen durch den Riegelkopf verschlossenen Sackbohrung 45 im Riegelbolzen 41 angeordnet ist und radial nach innen am Hülsenzapfen 38 oder an einem in die Sackbohrung 45 hineinragenden Stift 46 abgestützt ist. Der Riegelbolzen 41 ist vorzugsweise einstückig mit einem von seinem unteren Ende nach vorne ragenden stabförmigen Druckstück 47 vorzugsweise etwa rechteckigen Querschnitts verbunden, das in einer axialen Führungsnut im Hülsenzapfen 38 mit dem Riegelbolzen 41 radial verschiebbar gelagert ist. Die Führungsnut 48 ist in einem nach vorne gerichteten Abstand a vom Flansch 39 radial nach außen offen, wobei das Druckstück 47 in diesen Führungsnutabschnitt 48a mit einem radialen Druckstückansatz 47a radial nach außen eintaucht und in der ausgeschobenen Stellung mit seiner an die Krümmung der Mantelfläche 49 des Hülsenzapfens 38 angepaßten Außenfläche 47b mit der Mantelfläche 49 abschließt. Innerhalb der Abstands a ist auf dem Hülsenzapfen 38 in einer Umfangsnut ein Spannring 51 gelagert, der in seiner radial entspannten Stellung die Mantelfäche 49 mit einem Spannwulst geringfügig überragt. Die Führungsnut 48 ist teilweise im Hülsenzapfen 38 und teilweise in einer von hinten in den Hülsenzapfen 38 fest eingeschobenen Hülsenbuchse 52 ausgebildet, wobei die Führungsnut 48 radial innen durch einen Steg 52a der Hülsenbuchse 52 verschlossen ist. Der vordere Außenrand des Hülsenzapfens 38 bei 38a ist konisch geformt oder gerundet.

Der Kühlmittelschlauch 7a ist im Bereich des Steckers 9, vorzugsweise in einem rückseitigen verjüngten Stutzen 53 desselben schräg nach außen herausgeführt, wozu ein schräger Kanal 54 im Stutzen 53 dient, der vom vorhandenen zentralen Stutzenkanal 55 ausgeht.

Zur Weiterleitung des Kühlmediums vom Röhrchen 34 zum Werkzeugträgerteil 2b ist ein kleiner Schlauch 56 vorgesehen (Fig. 1) der rückseitig auf das Röhrchen 34 aufgesteckt ist und vorderseitig mit einem Schlauchanschluß, vorzugsweise ebenfalls in Form eines kleinen Röhrchens, vorzugsweise im vorderen Bereich des Werkzeugträgerteils 2b verbunden ist. Der schräg aus dem Stecker 9 herausgeführte Abschnitt des Kühlmittelschlauchs 7a ist mit seinem rückseitigen Ende an einen Leitungsanschluß der Pumpe 6 angeschlossen.

Zur Vergrößerung der Griffestigkeit ist das Antriebsteils 2a leicht ballig geformt. Vorzugsweise sind sein vorderes Drittel zylindrisch und sein übriger Abschnitt ballig geformt.

Bei der insbesondere chirurgischen Behandlung eines Körpers mit dem Behandlungswerkzeug 5 ist eine Kontaminierung mit Krankheitserregern des Handstücks 2 vorgegeben. Man ist deshalb dazu übergegangen, nicht nur das Behandlungswerkzeug 5 und das Werkzeugträgerteil 2b sondern aus das Antriebsteils 2a und die Versorgungsleitung 4 nach jeder Behandlung zu sterilisieren. Dies kann z.B. in einem Autoklaven bei einem Dampfdruck von etwa 3 bar und einer Temperatur von etwa 145°C oder in einer Sterillisationsflüssigkeit erfolgen. Für eine solche Sterillisationsbehandlung ist ein elektrischer Antriebsmotor bzw. eine Motorpatrone 14 nur mit sehr aufwendigen Abdichtungsmitteln, die darüber hinaus temperaturbeständig sein müssen, geeignet, deren Aufwand die Behandlungseinrichtung sehr verteuert. Es ist deshalb vorgesehen, die Motorpatrone 14 von einer Kontaminierung zu schützen und von der Sterillisationbehandlung freizustellen. Hierzu dient eine Parkstelle 61 mit einer Aufnahmevorrichtung 62 für die Motorpatrone 14 während der Sterillisation wenigstens des Behandlungswerkzeugs 5 und des Werkzeugträgerteils 2b und vorzugsweise auch des Antriebsteils 2a mit der Versorgungsleitung 4. Die Parkstelle 61 bzw. der zugehörige Parkplatz mit der Aufnahmevorrichtung 61 sind vorzugsweise am Steuergerät 3 angeordnet, so daß ein weiterer Gegenstand des vorhandenen Behandlungsplatzes nicht spezifisch ausgebildet zu werden braucht. Vorzugsweise ist die Parkstelle 61 mit der Aufnahmevorrichtung 62 an der Frontseite des Steuergeräts 3 angeordnet. Es ist auch vorteilhaft, die Aufnahmevorrichtung 62 an der Pumpe 6 oder einer sie tragenden Anschlußplatte 63 auszubilden, die zwischen dem Steuergerät 3 und der Pumpe 6, vorzugsweise seitlich am Steuergerät 3, angeordnet ist und insbesondere an seiner Vorderseite auch die Stockkupplung 8 für den Stecker 9 aufweisen kann. Bei der vorliegenden Ausgestaltung ist die Steckkupplung 8 unterhalb der Aufnahmevorrichtung 62 an der Anschlußplatte 63 angeordnet. Vorzugsweise ist die Aufnahmevorrichtung 62 durch eine Steckbuchse 64 mit einem von außen zugänglichen Steckloch 65 gebildet, die in der Anschlußplatte 63 angeordnet ist (siehe Figuren 6 und 7). Das Steckloch 65 ist so groß bemessen, daß der Hülsenzapfen 38 der Motorpatrone 14 mit Bewegungsspiel darin einsteckbar ist.

Der Aufnahmevorrichtung 62 ist ein Kupplungsmittel 66 zur lösbaren Kupplung der Motorpatrone 14 an die Aufnahmevorrichtung 62 und ein Entkupplungsbetätigungsmittel 67 zum Lösen der zwischen der Motorpatrone 14 und der Hülse 15 wirksamen und durch den Riegelbolzen 41 und die Ringnut 43 gebildeten ersten Kupplung 68.

Das Kupplungsmittel 66 wird durch eine Verrastungskante 69 am äußeren Ende der Steckbuchse 64 bzw. des Stecklochs 65 gebildet, hinter der einer Ringnut 71 in der Steckbuchse 64 angeordnet ist, in die gegebenenfalls der Spannring 51 mit seinem Ringwulst federnd und somit lösbar einzurasten vermag. Hierdurch wird eine zweite Kupplung 72 gebildet, die in der in die Aufnahmevorrichtung 62 eingesteckten Position der Motorpatrone 14 automatisch wirksam ist, und letztere an der Aufnahmevorrichtung 62 lösbar sichert.

Das Endkupplungsbetätigungsmittel 67 wird durch ein Druckglied 73 gebildet, das in einem solchen Abstand vom äußeren Ende in die Aufnahmevorrichtung bzw. Steckbuchse 64 integriert ist, daß es radial einwärts gegen das Druckstück 47 zu drücken vermag. Die Druckkraft des Druckgliedes 73 ist größer als die Kupplungsschließkraft der ersten Kupplung 68, d.h. größer als die Kraft der Feder 42, mit der der Riegelbolzen 41 und das Druckstück 47 in ihre Kupplungsstellung beaufschlagt sind.

Vorzugsweise ist das Druckglied 73 eine Kugel, die in einem etwas Kleiner als ihr Durchmesser bemessenen Loch 74 in der Steckbuchse 64 soweit radial einwärts beweglich ist, daß sie um ein solches Maß in das Steckloch 65 einzutauchen vermag, das größer ist, als die Endkupplungsbewegung des Druckstücks 47 bzw. des Ringbolzen 41. Bei der vorliegenden Ausgestaltung befindet sich das Loch 74 im Grund einer Außenumfangsnut 75 der Steckbuchse 64, in der ein Druckfederring 76 mit Bewegungsspiel angeordnet ist, der sich über das Druckglied 73 erstreckt und dieses radial einwärts elastische beaufschlagt.

Die vorbeschriebene erste Kupplung 68 zwischen dem Antriebsteil 2a und der Motorpatrone 14, die zweite Kupplung 72 zwischen der Motorpatrone 14 und der Aufnahmevorrichtung 62 und das Endkupplungsbetätigungsmittel 67 zum Lösen der ersten Kupplung 68 ermöglichen eine handhabungsfreundliche Deponierung der Motorpatrone 14 an der Parkstelle 61 zum Sterilisieren des Antriebsteils 2a mit seiner Versorgungsleitung 4 und auch eine handhabungsfreundliche Rückübergabe der Motorpatrone 4 an das Antriebsteil 2a. Bei dieser Übergabe und Rückübergabe braucht die Motorpatrone 14 nicht gesondert manuell angefaßt zu werden.

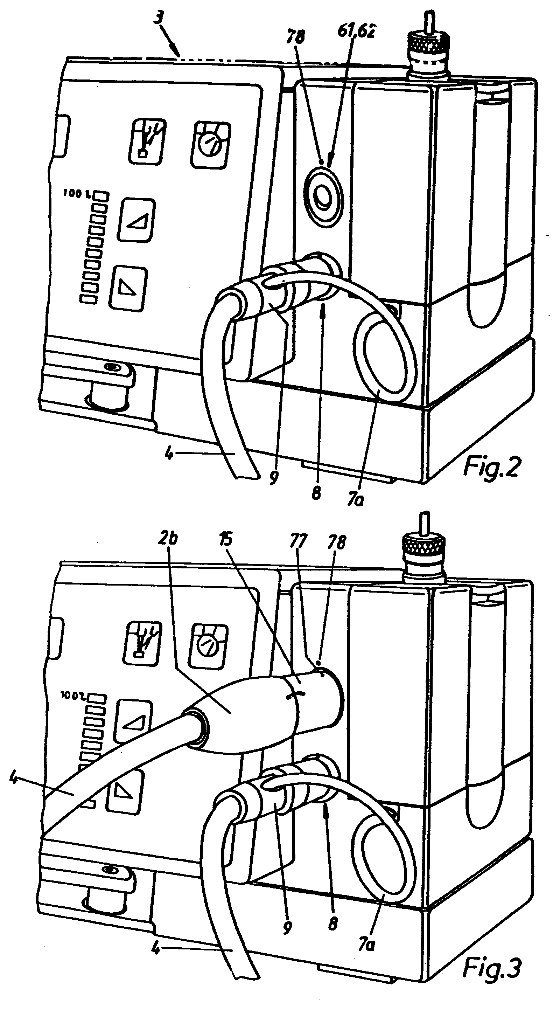

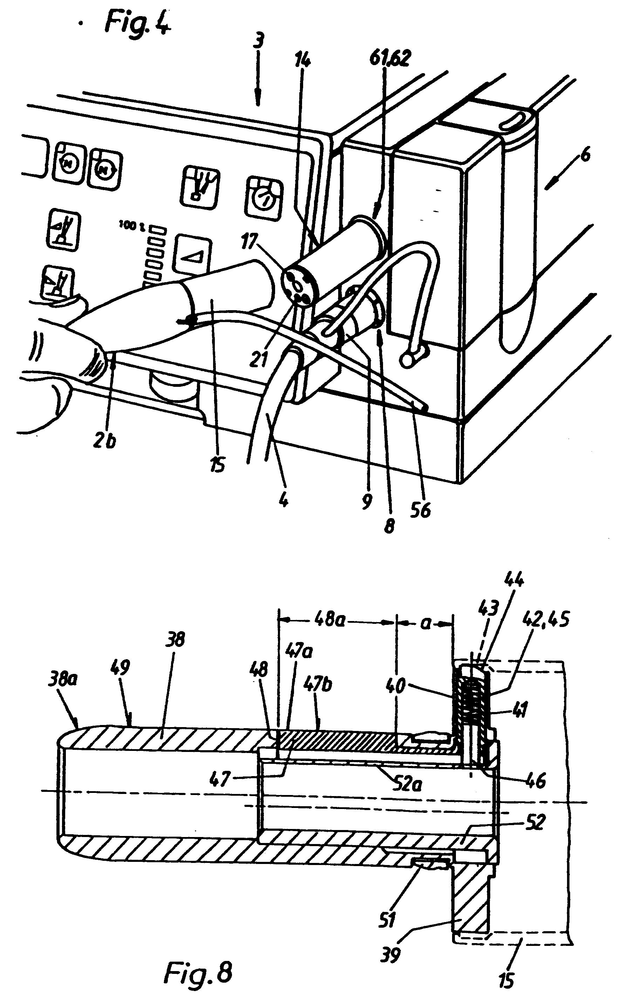

Im Folgenden wird eine solche Übergabe und Rückübergabe beschrieben. Nach der jeweiligen Behandlung Fig. 1 und 2 des zu behandelnden Körpers wird das Werkzeugträgerteil 2b vom Antriebsteil 2a entfernt, was durch Lösen einer Kupplung erfolgt, beispielsweise durch den Spannring 51 und einem dem Werkzeugträgerteil 2b zugeordneter entsprechenden Kupplungselement. Das Werkzeugträgerteil 2b wird manuell abgenommen und einer Sterilisation zugeführt. Das Antriebsteils 2a wird mit der darin befindlichen und gekuppelten Motorpatrone 14 zur Parkstelle 61 bewegt und der Hülsenzapfen 38 wird in das Steckloch 65 eingesteckt, so daß die zweite Kupplung 72 schließt und somit die Motorpatrone 14 gegen unbeabsichtigtes Herausziehen aus der Aufnahmevorrichtung 62 gesichert ist (Fig. 39). Wenn das Druckglied 73 des Endkupplungsbetätigungsmittels 67 auf das Druckstück 47 trifft, wird die erste Kupplung 68 zwischen der Motorpatrone 14 und der Hülse 15 automatisch gelöst, so daß das Antriebsteil 2a mit seiner Hülse 15 von der in der Aufnahmevorrichtung 62 gehaltenen Motorpatrone 14 abgezogen werden kann (Fig. 4.). Vorzugsweise wird die Motorpatrone 14 in einer solchen Drehstellung in die Aufnahmevorrichtung 62 eingesteckt, in der das Druckglied 73 selbsttätig auf das Druckstück 47 gelagt. Diese Drehstellung kann durch eine Markierung 77 am Umfang der Hülse 15 und in deren vorderen Bereich und durch eine Markierung 78 an der Aufnahmevorrichtung 62 bzw. die Anschlußplatte 63 oberhalb der Steckbuchse 64 gebildet sein. Es jedoch auch möglich, die Motorpatrone 14 in einer drehversetzten Position einzustecken und durch Drehen das Druckglied 73 und das Druckstück 47 in Kontakt miteinander zu bringen.

Die Rückübergabe der Motorpatrone 14 an das Antriebsteil 2a läßt sich nach der Sterilisation ebenfalls handhabungsfreundlich dadurch bewerkstelligen, daß das Antriebsteil 2a mit seiner Hülse 15 auf die Motorpatrone 14 soweit aufgesteckt wird, daß die zwischen dem Antriebsteil 2a und dem freien Stirnende der Motorpatrone 14 wirksame und durch die Stifte 17, 21 gebildete Drehkupplung 79 geschlossen wird Fig. 7. Danach wird das Antriebsteil 2a mit der Motorpatrone 14 in eine der beiden wahlweisen Drehrichtungen gedreht, wodurch das Druckglied 73 und das Druckstück 47 außer Eingriff gelangen und das Druckstück 47 mit dem Riegelbolzen 41 automatisch in ihre Kupplungsstellung gelangen, wodurch die erste Kupplung 68 geschlossen wird. Danach kann durch die Zugkraft beim axialen Herausziehen der Motorpatrone 14 aus der Aufnahmevorrichtung 62 die zweite Kupplung 72 leicht gelöst und das Antriebsteil 2a mit der Motorpatrone 14 von der Parkstelle 61 entfernt werden (Fig. 6). Danach bedarf es lediglich noch des Anbaus des Werkzeugträgerteils 2b, um eine erneute Behandlung durchzuführen.

Der Schutz der Motorpatrone 14 vor Kontamination ist unter anderem dadurch gesichert, daß sie im Handstück 2 völlig gekapselt ist und während der Sterillisation des Handstücks 2 an der Parkstelle 61 deponiert werden kann.

Aufgrund der vorhandenen Schnellkupplung oder Steckfassung zwischen dem Ablageteil 13 und dem Tragarm 12a läßt sich das Ablageteil 13 leicht und schnell demontieren bzw. montieren, insbesondere zwecks Sterilisation ggfs. mit dem Handstück 2 und der Versorgungsleitung 4.

Die Behandlungseinrichtung eignet sich insbesondere für die chirurgische oder microchirurgische Behandlung menschlicher oder tierischer Körper.

## Patentansprüche

1. Ärztliche Behandlungseinrichtung (1) für einen Behandlungsplatz, insbesondere für chirurgische Zwecke, die ein motorbetriebenes Behandlungsinstrument aufweist mit einem aus mindestens zwei Teilen bestehenden Handstück (2), von denen das hintere Handstückteil (2a) durch eine Medien-Versorgungsleitung (4) mit einem Steuergerät (3) verbindbar ist und das vordere Handstückteil (2b) zur Aufnahme eines Behandlungswerkzeugs (5) dient, wobei eines der beiden Handstückteile (2a, 2d) eine separierbare Motorpatrone (14) enthält, wobei eine erste Kupplung (68) mit Kupplungsmitteln (41, 43) zum lösbaren Kuppeln des zugehörigen Handstückteils (2a) und der Motorpatrone (14) vorgesehen ist, wobei im Bereich des Behandlungsplatzes eine Parkstelle (61) mit einer Aufnahmevorrichtung (62) für die Motorpatrone (14) vorgesehen ist, der die Motorpatrone (14) mit dem sie tragenden Handstückteil (2a) übertragbar ist, und von der die Motorpatrone (14) mit dem sie tragenden Handstückteil (2a) wieder rückübertragbar ist, und wobei eine zweite Kupplung (72) mit Kupplungsmitteln (51, 69) zum lösbaren Kuppeln der Motorpatrone (14) und der Aufnahmevorrichtung (62) in der auf sie übertragenen Position vorgesehen ist.

2. Behandlungseinrichtung nach Anspruch 1,
**dadurch gekennzeichnet,**
daß das die Versorgungsleitung (4) aufweisende Handstückteil (2a) die Motorpatrone (14) enthält.

3. Behandlungseinrichtung nach Anspruch 2,
**dadurch gekennzeichnet,**
daß das eine Handstückteil (2a) eine dem anderen Handstückteil (2b) zugewandte, Hülse (15) aufweist, die zumindest den Motorkörper der Motorpatrone (14) überdeckt.

4. Behandlungseinrichtung nach Anspruch 3,
**dadurch gekennzeichnet,**
daß die Hülse (15) am hinteren Handstückteil (2a) angeordnet ist und sich von diesem nach vorne erstreckt.

5. Behandlungseinrichtung nach einem der vorherigen Ansprüche,
**dadurch gekennzeichnet,**
daß der Aufnahmevorrichtung (62) ein Entkupplungsbetätigungsmittel (67) zum Lösen der Kupplung (68) in der der Aufnahmevorrichtung (67) übertragenen Position der Motorpatrone (14) zugeordnet ist.

6. Behandlungseinrichtung nach wenigstens einem der vorherigen Ansprüche,
**dadurch gekennzeichnet,**
daß die Aufnahmevorrichtung (62) durch eine Steckbuchse (64) gebildet ist.

7. Behandlungseinrichtung nach Anspruch 6,
**dadurch gekennzeichnet,**
daß die Steckbuchse (64) horizontal angeordnet ist.

8. Behandlungseinrichtung nach einem der vorherigen Ansprüche,
**dadurch gekennzeichnet,**
daß die Motorpatrone (14) an ihrem vorderen Ende einen Steckzapfen (38) aufweist, mit dem sie in die Aufnahmevorrichtung (62) einsetzbar oder in die Steckbuchse (64) einsteckbar ist.

9. Behandlungseinrichtung nach einem der vorherigen Ansprüche,
**dadurch gekennzeichnet,**
daß die erste Kupplung (68) ein Lösungs-Betätigungsglied (47) aufweist, das von vorne zugänglich ist.

10. Behandlungseinrichtung nach Anspruch 9,
**dadurch gekennzeichnet,**
daß das Lösungs-Betätigungsglied (47) sich innerhalb des Steckzapfens (38) vom Motor-Patronenkörper nach vorne erstreckt.

11. Behandlungseinrichtung nach einem der vorherigen Ansprüche,
**dadurch gekennzeichnet,**
daß die Aufnahmevorrichtung (62) am Steuergerät (3) angeordnet ist.

12. Behandlungseinrichtung nach Anspruch 11,
**dadurch gekennzeichnet,**
daß die Aufnahmevorrichtung (62) an der Frontseite des Steuergeräts (3) angeordnet ist.

## Claims

1. Medical treatment device (1) for a treatment station, in particular for surgical purposes, which comprises a motor-operated treatment instrument having a handle (2) made up of at least two parts, of which the rear handle part (2a) is connectable by a medium supply line (4) to a control unit (3) and the front handle part (2b) is used to receive a treatment tool (5), wherein one of the two handle parts (2a, 2b) contains a separable motor cartridge (14), wherein a first coupling (68) with coupling means (41, 43) is provided for releasable coupling of the associated handle part (2a) and the motor cartridge (14), wherein in the region of the treatment station a parking place (61) having a receiving device (62) for the motor cartridge (14) is provided, to which the motor cartridge (14) with its carrying handle part (2a) is transferable and from which the motor cartridge (14) with its carrying handle part (2a) is re-transferable, and wherein a second coupling (72) having coupling means (51, 69) is provided for releasable coupling of the motor cartridge (14) and the receiving device (62) in the position of transfer onto said receiving device.

2. Treatment device according to claim 1,
**characterized in**
that the handle part (2a) having the supply line (4) contains the motor cartridge (14).

3. Treatment device according to claim 2,
**characterized in**
that the one handle part (2a) has a sleeve (15), which is directed towards the other handle part (2b) and covers at least the motor body of the motor cartridge (14).

4. Treatment device according to claim 3,
**characterized in**
that the sleeve (15) is disposed on the rear handle part (2a) and extends forward from the latter.

5. Treatment device according to one of the previous claims,
**characterized in**
that associated with the receiving device (62) is an uncoupling means (67) for releasing the coupling (68) in the position of transfer of the motor cartridge (14) to the receiving device (67).

6. Treatment device according to at least one of the previous claims,
**characterized in**
that the receiving device (62) is formed by a receptacle (64).

7. Treatment device according to claim 6,
**characterized in**
that the receptacle (64) is disposed horizontally.

8. Treatment device according to one of the previous claims,
**characterized in**
that the motor cartridge (14) at its front end has a plug-in pin (38), by means of which it is insertable into the receiving device (62) or into the receptacle (64).

9. Treatment device according to one of the previous claims,
**characterized in**
that the first coupling (68) has a releasing element (47), which is accessible from the front.

10. Treatment device according to claim 9,
**characterized in**
that the releasing element (47) extends inside the plug-in pin (38) forward from the motor cartridge body.

11. Treatment device according to one of the previous claims,
**characterized in**
that the receiving device (62) is disposed on the control unit (3).

12. Treatment device according to claim 11,
**characterized in**
that the receiving device (62) is disposed at the front of the control unit (3).

## Revendications

1. Dispositif de traitement (1) médical pour un poste de traitement, notamment pour des applications chirurgicales, comportant un instrument de traitement actionné par moteur avec une pièce à main (2) en au moins parties, parmi lesquelles la partie arrière (2a) de pièce à main est connectée à un appareil de commande (3) par l'intermédiaire d'une conduite d'alimentation en fluide (4) et la partie avant (2b) de pièce à main sert à recevoir un outil de traitement (5), l'une des deux parties de pièce à main (2a, 2d) contenant une cartouche moteur (14) séparable, un premier dispositif d'accouplement (68) avec des moyens d'accouplement (41, 43) étant prévu pour accoupler de manière séparable la partie de pièce à main (2a) concernée avec la cartouche moteur (14), un emplacement de rangement (61) avec un dispositif de réception (62) pour la cartouche moteur (14) étant prévu dans la zone du poste de traitement, emplacement où la cartouche moteur (14) avec la partie de pièce à main (2a) qui la porte peut être déposée ou reprise, et un deuxième dispositif d'accouplement (72) avec des moyens d'accouplement (51, 69) étant prévu pour accoupler de manière séparable la cartouche moteur (14) avec le dispositif de réception (62) dans la position transmise.

2. Dispositif de traitement selon la revendication 1, caractérisé par le fait que la partie de pièce à main (2a) pourvue de la conduite d'alimentation (4) contient la cartouche moteur (14).

3. Dispositif de traitement selon la revendication 2, caractérisé par le fait que l'une des parties de pièce à main (2a) comporte un manchon (15) tourné vers l'autre partie de pièce à main (2b), qui couvre au moins partiellement le corps de moteur de la cartouche moteur (14).

4. Dispositif de traitement selon la revendication 3, caractérisé par le fait que le manchon (15) est disposé sur la partie de pièce à main (2a) arrière et s'étend vers l'avant à partir de celle-ci.

5. Dispositif de traitement selon une des revendications précédentes, caractérisé par le fait qu'un moyen d'actionnement de désaccouplement (67) pour libérer le dispositif d'accouplement (68) dans la position de la cartouche moteur (14) transmise au dispositif de réception (62) est associé au dispositif de réception (62).

6. Dispositif de traitement selon au moins une des revendications précédentes, caractérisé par le fait que le dispositif de réception (62) est formé d'une douille à enfichage (64).

7. Dispositif de traitement selon la revendication 6, caractérisé par le fait que la douille à enfichage (64) est disposée horizontalement.

8. Dispositif de traitement selon une des revendications précédentes, caractérisé par le fait que la cartouche moteur (14), à son extrémité antérieure, présente un embout enfichable (38) à l'aide duquel elle peut être placée dans le dispositif de réception (62) ou enfichée dans la douille à enfichage (64).

9. Dispositif de traitement selon une des revendications précédentes, caractérisé par le fait que le premier dispositif d'accouplement (68) comporte un élément d'actionnement de séparation (47) qui est accessible par le devant.

10. Dispositif de traitement selon la revendication 9, caractérisé par le fait que l'élément d'actionnement de séparation (47) s'étend à l'intérieur de l'embout enfichable (38), vers l'avant à partir du corps de cartouche moteur.

11. Dispositif de traitement selon une des revendications précédentes, caractérisé par le fait que le dispositif de réception (62) est disposé sur l'appareil de commande (3).

12. Dispositif de traitement selon la revendication 11, caractérisé par le fait que le dispositif de réception (62) est disposé sur la face avant de l'appareil de commande (3).
